# EUROPÄISCHE PATENTANMELDUNG

(11) **EP 4 246 132 A2**
(43) Veröffentlichungstag der Anmeldung: **20.09.2023**
(21) Anmeldenummer: 23191073.8
(22) Anmeldetag: 19.02.2020
(51) Int. Cl.: G01N 21/94

(54) **KONTINUIERLICHE PRESSE MIT EINER VORRICHTUNG ZUR ÜBERWACHUNG DES SCHMIERZUSTANDES EINES MIT EINEM SCHMIERMITTEL BEAUFSCHLAGTEN UMLAUFENDEN BANDES**

(30) Priorität: 20.03.2019 DE 102019107152
(62) Teilanmeldung aus: 20707029.3
(71) Anmelder: Siempelkamp Maschinen- und Anlagenbau GmbH, 47803 Krefeld (DE)
(72) Erfinder: Gartz, Klaus, 41749 Viersen (DE)
(74) Vertreter: Andrejewski - Honke Patent- und Rechtsanwälte Partnerschaft mbB

(57) **Zusammenfassung**

Die Erfindung betrifft eine kontinuierliche Presse mit einer Vorrichtung zur Überwachung des Schmierzustandes eines mit einem Schmiermittel beaufschlagten, umlaufenden Bandes (4) für den Transport von Pressgut in der kontinuierlich arbeitenden Presse (1), mit zumindest einer im Bereich des Bandes (4) angeordneten ersten Detektionseinrichtung (D1) eines ersten Typs und mit zumindest einer im Bereich des Bandes (4) angeordneten zweiten Detektionseinrichtung (D2) eines zweiten Typs.

Die Überwachungsvorrichtung ist dadurch gekennzeichnet, dass die erste Detektionseinrichtung (D1) dazu eingerichtet ist, ein von der Menge des auf dem Band (4) befindlichen Materials abhängiges Mengensignal zu erzeugen und dass zur Unterscheidung eines auf dem Band (4) befindlichen Schmiermittels von auf dem Band (4) befindlichen Schmutzpartikeln oder von einem mit Schmutzpartikeln verunreinigten Schmiermittel die zweite Detektionseinrichtung (D2) dazu eingerichtet ist, ein von der Art des auf dem Band (4) befindlichen Materials abhängiges Materialsignal ( S2) zu erzeugen.

## Beschreibung

Die Erfindung betrifft eine kontinuierliche Presse, mit einem oberen, endlos umlaufenden Band bzw. Pressband und einem unteren endlos umlaufenden Band bzw. Pressband, wobei Pressgut durch den Pressspalt zwischen dem oberen und dem unteren Band hindurchgeführt und unter Anwendung von Druck und/oder Wärme zu einer Platte bzw. einem endlosen Plattenstrang verpresst wird. Die endlos umlaufenden Bänder einer solchen Presse werden auch als Pressbänder bezeichnet und sie sind bevorzugt als Stahlbänder ausgebildet. Außerdem betrifft die Erfindung ein Verfahren zum Betreiben einer kontinuierlich arbeitenden Presse.

Die Erfindung betrifft auch eine Vorrichtung zur Überwachung des Schmierzustandes eines mit einem Schmiermittel beaufschlagten, umlaufenden Bandes für den Transport von Pressgut, in einer kontinuierlich arbeitenden bzw. kontinuierlich betreibbaren Presse, mit zumindest einer im Bereich des Bandes angeordneten ersten Detektionseinrichtung eines ersten Typs und mit zumindest einer im Bereich des Bandes angeordneten zweiten Detektionseinrichtung eines zweiten Typs.

Die Erfindung betrifft auch ein Verfahren zur Überwachung eines mit einem Schmiermittel beaufschlagten, umlaufenden Bandes für den Transport von Pressgut, in einer kontinuierlich arbeitenden Presse, wobei mit zumindest einer im Bereich des Bandes angeordneten ersten Detektionsvorrichtung eines ersten Typs und mit zumindest einer im Bereich des Bandes angeordneten zweiten Detektionsvorrichtung eines zweiten Typs zumindest ein Signal erzeugt wird.

Bevorzugt handelt es sich bei einer solchen kontinuierlich betreibbaren Presse um eine Doppelbandpresse, bei der im Pressenoberteil eine obere (beheizbare) Pressenplatte und im Pressenunterteil eine untere (beheizbare) Pressenplatte vorgesehen sind und wobei im Pressenoberteil das endlos umlaufende obere Pressband und im Pressenunterteil das endlos umlaufende untere Pressband geführt sind. Die Pressbänder sind bevorzugt unter Zwischenschaltung von Wälzkörpern, z. B. Rollstäben oder Rollstangen, an den Pressenplatten abgestützt. Die erforderlichen Presskräfte werden mit Kraftmitteln, insbesondere mit hydraulischen Presszylindern aufgebracht, mit denen z. B. die obere und/oder die untere Pressenplatte beaufschlagt wird und die sich an einem Pressengestell, z. B. an den Pressenrahmen eines Pressengestells abstützen. Die Rollstäbe sind z. B. an Rollstabketten angeschlossen und mit Hilfe solcher Rollstabketten im Umlauf geführt.

Eine solche Presse, die auch kontinuierlich arbeitende Presse genannt wird, wird z. B. für die Herstellung von Holzwerkstoffplatten eingesetzt. Holzwerkstoffplatten meint insbesondere Faserplatten, Spanplatten oder OSB-Platten. Alternativ betrifft die Erfindung aber auch derartige Pressen für die Herstellung von Verbundplatten oder von Verbundbauteilen, z. B. aus Faserverbundwerkstoffen oder dergleichen oder auch von Kunststoffplatten oder Kunststoffmatten.

Das Pressgut (z. B. eine Pressgutmatte) wird im Pressspalt der kontinuierlich arbeitenden Presse zwischen den umlaufenden Stahlbändern bzw. Pressbändern unter Anwendung von Druck- und Wärme verpresst, so dass aus dem Pressgut z. B. ein plattenförmiges Produkt, z. B. eine Holzwerkstoffplatte hergestellt wird. Dabei rollen die Rollstäbe an den Pressenplatten als Wälzkörperaggregate ab. Aufgrund üblicher Fertigungstoleranzen bzw. minimalsten Abweichungen der Rollstabform von der perfekten Zylinderform kann es im Zuge der Abrollbewegung zu Weg- bzw. Längenunterschieden an den beiden äußeren Enden der Rollstäbe kommen, die durch einen Mikroschlupf kompensiert werden. Vor diesem Hintergrund ist es in der Praxis üblich und technisch erforderlich, die Rollstäbe bzw. deren zylindrische Oberfläche und/oder die korrespondierende Fläche des umlaufenden Bandes mit einem Schmiermittel zu schmieren. Ein solches Schmiermittel ist bevorzugt als flüssiges Schmiermittel (z. B. Öl) ausgebildet und es wird in der Praxis z. B. mit Hilfe von Düsen oder dergleichen im Einlaufbereich oder im Rollstabumlauf auf die Rollstäbe und/oder auf die Pressbänder aufgebracht, z. B. aufgesprüht. Es erfolgt demnach eine Beaufschlagung der den Rollstangen zugewandten (inneren) Oberfläche des endlos umlaufenden Bandes mit einem Schmiermittel, und zwar entweder unmittelbar durch z. B. Bedüsung der Bandoberfläche oder mittelbar über die mit dem Schmiermittel beaufschlagten (z. B. bedüsten) Rollstangen.

Für den einwandfreien Betrieb einer kontinuierlich betreibbaren Presse ist eine einwandfreie Schmierung von besonderer Bedeutung, wobei in der Regel einerseits eine Unterschmierung und andererseits eine Überschmierung verhindert werden soll. Es ist daher in der Praxis vorgesehen, den Schmierzustand bzw. die durch die Bedüsung aufgebrachte Schmiermittel- bzw. Ölmenge zu überwachen, und zwar in der Regel manuell bzw. visuell durch das Servicepersonal aufgrund von Erfahrungswerten. Dabei soll darauf geachtet werden, dass sich die auf die Bandoberfläche zur Schmierung aufgebrachte Schmiermittelmenge gleichmäßig verteilt und keine Bereiche entstehen, in denen die Schmierung aufgrund von Schmiermittelmangel, z. B. Ölmangel nicht ausreichend bemessen ist. Grundsätzlich haben sich diese einfachen Maßnahmen in der Praxis bewährt. Es besteht jedoch das Bedürfnis die Überwachung zu optimieren und insbesondere zu automatisieren. So besteht insbesondere das Bedürfnis, eine zuverlässige und quantifizierbare Überwachung einzurichten, um einerseits zuverlässig Mangelschmierungen zu verhindern und andererseits den Schmiermittelverbrauch z. B. aus wirtschaftlichen Gründen innerhalb der erforderlichen Grenzen zu halten.

Kontinuierlich arbeitende Pressen mit zugehörigen Schmiersystemen sind z. B. aus der DE 31 48 412 A1, DE 40 15 706 A1 sowie DE 41 26 717 C1 bekannt.

Aus der DE 10 2016 102 931 B4 ist ferner eine Vorrichtung zur Überwachung und/oder Regelung eines Schmierzustandes in einer kontinuierlich arbeitenden Presse bekannt, bei der ein oder mehrere Sensoren zum Messen eins physikalischen Phänomens vorgesehen sind, das mit einer Schmiermittelmenge auf dem Band zusammenhängt. Dazu soll eine Auswerteeinheit zum Ermitteln eines Schmierparameters als Kenngröße für eine Schmiermittelmenge auf dem Band basierend auf dem Messwert des Sensors vorgesehen sein. Als Sensor kann z. B. ein Lichtsensor zum Messen eines Lichtreflexionswertes des Bandes und/oder einer Rollstange oder ein Lichtsensor zum Messen eines Lichtabsorptionswertes des Bandes und/oder einer Rollstange der Presse sein. Alternativ können ein Leitfähigkeitssensor, ein Laserabstandsensor oder ein Ultraschallsensor zum Einsatz kommen. Des Weiteren werden Druck-, Zug-, Leistungs- und/oder Kraftsensoren erwähnt. Es soll eine steuerbare Schmiereinheit zum Aufbringen von Schmiermittel vorgesehen sein, die mit einer Regeleinrichtung zur Regelung einer von der Schmiereinheit aufgebrachten Schmiermittelmenge basierend auf dem ermittelten Schmierparameter eingerichtet ist. Im Übrigen soll die Möglichkeit bestehen, Signale mehrerer gleicher oder verschiedener Sensoren zu kombinieren.

Im Übrigen kennt man aus der WO 2016/037735 A1 ein Verfahren zur Ermittlung des Zustandes einer Transportbandoberfläche an einem bewegten Transportband mithilfe einer Polarisationskamera. Mit dem Verfahren soll es möglich sein, den Oberflächenzustand eines Transportbandes aufgrund des in den Polarisationsebenen auftretenden Kontrastes zwischen den einzelnen unterschiedlich beanspruchten Transportbandabschnitten zu ermitteln. So sollen Wartung-, Pflege- oder auch Reparaturmaßnahmen am Transportband durchgeführt werden, sobald vollflächige oder abschnittsweise Veränderungen des Oberflächenzustandes ermittelt werden.

Schließlich beschreibt die WO 99/31459 A1 ein Verfahren und eine Vorrichtung zur berührungslosen Feststellung oder Überprüfung eines Fluidauftrages, insbesondere eines Leim-, Kleber-, Lack- oder Kunststoffauftrages, auf einer Oberfläche eines Werkstückes, wobei sowohl vor als auch nach einer Fluidauftragseinheit zumindest ein Messsensor mit zumindest einer Elektrode angeordnet ist. Die Messsensoren sind als kapazitive Messsensoren ausgebildet.

Ausgehend von dem vorbekannten Stand der Technik liegt der Erfindung das technische Problem zugrunde, eine Presse bzw. eine Vorrichtung sowie ein Verfahren zu schaffen, mit der bzw. mit dem sich der Schmierzustand eines umlaufenden Bandes, in einer kontinuierlich arbeitenden Presse, in wirtschaftlicher Weise besonders zuverlässig überwachen lässt.

Zur Lösung dieser Aufgabe lehrt die Erfindung bei einer gattungsgemäßen Presse oder Vorrichtung der eingangs beschriebenen Art, dass einerseits die erste Detektionseinrichtung dazu eingerichtet ist, ein von der Menge des auf dem Band befindlichen Materials abhängiges Mengensignal zu erzeugen und dass andererseits zur Unterscheidung eines auf dem Band befindlichen Schmiermittels von auf dem Band befindlichen Schmutzpartikeln oder von einem mit Schmutzpartikeln verunreinigten Schmiermittel die zweite Detektionseinrichtung dazu eingerichtet ist, ein von der Art des auf dem Band befindlichen Materials abhängiges Materialsignal zu erzeugen.

Die Erfindung geht dabei von der Erkenntnis aus, dass eine besonders zuverlässige Überprüfung bzw. Überwachung des Schmierzustandes eines umlaufenden Bandes durch Einsatz mehrerer Detektionseinrichtungen mit unterschiedlichen Detektionscharakteristiken möglich ist, indem nämlich die erste Detektionseinrichtung auf die mengenmäßige bzw. mengenabhängige Erfassung des Schmiermittels ausgerichtet ist und die zweite Detektionseinrichtung materialsensitiv arbeitet und der Unterscheidung verschiedener Materialien dient. Im Vordergrund steht dabei die Unterscheidung eines sauberen Schmiermittels (z. B. Öl) und folglich eines Schmiermittels ohne übermäßige Verschmutzung durch Fremdpartikel von einem übermäßig verschmutzten Schmiermittel oder auch von Partikeln selbst, z. B. Holzpartikel. Zugleich soll die Erfindung jedoch auch eine mengenmäßige Erfassung des Schmiermittels und folglich eine mengenmäßige Überprüfung des Schmierzustandes ermöglichen, so dass z. B. eine Mangelschmierung einerseits und auch eine (unwirtschaftliche) Überschmierung andererseits quantitativ bestimmt oder zumindest erkannt werden kann. Die Ermittlung bzw. Angabe einer Materialmenge als Messsignal muss dabei nicht eine tatsächliche, absolute Mengenangabe sein, sondern das Mengensignal kann sich auf eine relative Angabe bzw. Information beschränken, z. B. derart, dass angegeben wird, ob eine Schmiermittelmenge innerhalb eines vorgegebenen Bereiches liegt oder nicht. Von besonderer Bedeutung ist die Tatsache, dass mit der ersten Detektionseinrichtung zuverlässig eine quantitative Analyse bzw. Aussage und folglich eine Bestimmung der Menge des auf dem Band befindlichen Materials möglich ist, ohne dass mit dieser Detektionseinrichtung Rückschlüsse auf das auf dem Band befindliche Material möglich sein müssen. Insofern kann die erste Detektionseinrichtung maßgeblich auf eine besonders zuverlässige und/oder einfache mengenmäßige bzw. mengenabhängige Erfassung des Schmiermittels ausgelegt sein, ohne dass dabei der Verschmutzungsgrad des Schmiermittels erkannt werden muss. Demgegenüber kann die zweite Detektionseinrichtung auf eine optimale Materialunterscheidung ausgerichtet sein, ohne dass mit der zweiten Detektionseinrichtung bzw. dem eigesetzten Verfahren eine Aussage über die Menge möglich ist bzw. möglich sein muss.

Erfindungsgemäß kann die Überprüfung des Schmierzustandes folglich automatisiert oder zumindest maschinell unterstützt werden, so dass keine subjektive Beurteilung durch einen Operateur erfolgen muss, und zwar weder bezogen auf die Schmiermittelmenge noch bezogen auf die Schmiermittelqualität. Denn die erfindungsgemäße Vorrichtung kann sowohl ein (elektronisches) Mengensignal erzeugen als auch ein (elektronisches) Materialsignal, die z. B. in geeigneten Melde- und/oder Rechnersystemen visualisierbar und gegebenenfalls auch weiterverarbeitbar sind, z. B. im Rahmen einer Steuerung der Presse und insbesondere im Rahmen der Steuerung des Schmiermittelauftrags. So kann die Vorrichtung ein Melde- und/oder Rechnersystem aufweisen, mit dem das Mengensignal und/oder das Materialsignal anzeigbar sind, und zwar akustisch und/oder optisch. Alternativ oder ergänzend kann mit dem Melde- und/oder Rechnersystem das Mengensignal und das Materialsignal (gemeinsam) derart ausgewertet werden, dass aus dem Materialsignal alleine oder aus einer Kombination aus Materialsignal und/oder Mengensignal die Art des auf dem Band befindlichen Materials ermittelt werden kann. Insofern kann es sich zum einen um ein Meldesystem handeln, das insbesondere der Visualisierung der bereits vorliegenden Signale dient. Alternativ kann in diesem System jedoch auch eine Auswertung der Signale zum Zwecke der Unterscheidung unterschiedlicher Materialien erfolgen.

Eine mögliche Ausführungsform für die erste Detektionseinrichtung, die der quantitativen Analyse oder Erfassung des Schmiermittels und folglich der Erzeugung eines Mengensignals dient, ist eine Detektionseinrichtung, die auf kapazitiver Grundlage arbeitet und die eine oder mehrere kapazitiv arbeitende Sensoren aufweist, die jeweils zumindest eine in einem Abstand zum Band angeordnete Fühlerplatte aufweisen, die mit dem Band jeweils einen Kondensator bilden, dessen Kapazität von der auf dem Band angeordneten Menge des Materials, insbesondere Schmiermittels, abhängt.

Eine solche Ausgestaltung der Erfindung geht von der Erkenntnis aus, dass sich die auf der Bandoberfläche befindliche Schmiermittelmenge besonders einfach und zuverlässig mit kapazitiven Mitteln überwachen bzw. bestimmen lässt, und zwar durch Einbeziehung des aus einem elektrisch leitfähigen Material hergestellten Bandes, z. B. Stahlbandes. Es werden eine oder mehrere Fühlerplatten in einem vorgegebenen festen Abstand zu dem Band angeordnet, so dass die Fühlerplatte mit dem Band einen Kondensator bildet, dessen Kapazität empfindlich durch die dielektrische Wirkung des Schmiermittels, z. B. Öls, beeinflusst wird. Denn die Permitivität bzw. relative Dielektrizitätskonstante Er des Schmiermittels, z. B. Öls weicht mit einem Wert von etwa 2 sehr deutlich von der der Luft mit einem Wert von etwa 1 ab. Da diese Permitivität aufgrund des Zusammenhangs C=ε × εᵣ × A/d linear in die Kapazität des von Fühlerplatte und Band gebildeten Plattenkondensators eingeht, ist die Kapazität des auf diese Weise mit dem Band (z. B. Pressband einer kontinuierlichen Presse) gebildeten Kondensators ein empfindliches und zuverlässiges Maß für die auf dem Band und folglich innerhalb des Plattenkondensators angeordnete Schmiermittelmenge. Dabei kann ein grundsätzlich bekanntes Band bzw. Pressband aus elektrisch leitfähigem Material, z. B. ein Stahlband verwendet werden. Es ist jedoch ebenso möglich, ein Band, z. B. Pressband zu verwenden, das lediglich eine elektrisch leitfähige Oberfläche aufweist, die mit dem Schmiermittel benetzt ist.

Kapazitive Sensoren, die auf Basis der Veränderung der elektrischen Kapazität eines Kondensators arbeiten, sind zwar aus dem Stand der Technik bekannt, z. B. als Drucksensoren, Abstandssensoren, Näherungsschalter, Spaltsensoren, Wegsensoren oder auch als Feuchtigkeitssensoren. Für den Einsatz in kontinuierlichen Pressen und insbesondere für die quantitative Überwachung des Schmierzustandes von mit Schmiermittel beaufschlagten umlaufenden Bändern ist eine solche Technologie jedoch bislang nicht in Betracht gezogen worden.

In besonders vorteilhafter Weise besteht bei der ersten Detektionseinrichtung die Möglichkeit, den Schmierzustand des Bandes selektiv bzw. individuell über die Bandbreite zu analysieren bzw. zu überwachen, so dass nicht nur ein über die Breite gemittelter Schmierzustand erfasst werden kann, sondern insbesondere auch eine über die Breite ungleichmäßige Verteilung erfasst wird. Dieses gelingt bei der ersten Detektionseinrichtung z. B. mit den kapazitiv arbeitenden Sensoren. Denn in bevorzugter Weiterbildung wird vorgeschlagen, dass über die Breite des Bandes nebeneinander mehrere Fühlerplatten angeordnet sind, die mit dem Band nebeneinander mehrere Kondensatoren bilden, die jeweils unterschiedlichen Breitenpositionen des Bandes zugeordnet sind. Dabei besteht z. B. die Möglichkeit, über die Breite des Bandes zumindest fünf Fühlerplatten und folglich fünf Kondensatoren zu realisieren, so dass eine Überwachung des Schmierzustandes in fünf nebeneinander angeordneten Spuren möglich ist. Durch Aufteilung und Anzahl der Spuren kann eine Anpassung an die jeweiligen Gegebenheiten erfolgen, und zwar derart, dass durch eine höhere Anzahl von Spuren eine feinere bzw. selektivere Überwachung des Bandes über die Bandbreite ermöglicht wird.

Interessant ist die Tatsache, dass das Band, z. B. das Pressband einer kontinuierlichen Presse in der Regel ohnehin aus einem elektrisch leitfähigen Material gefertigt ist (z. B. als Stahlband) und dass ein solches Band üblicherweise ohnehin geerdet ist, so dass auf einfache Weise mit den Fühlerplatten ein oder mehrere Kondensatoren für eine kapazitive Schmiermittelüberwachung realisiert werden können.

In bevorzugter Weiterbildung einer z. B. als kapazitiv arbeitend ausgebildeten ersten Detektionseinrichtung ist vorgesehen, dass der oder die Sensoren (jeweils) eine (elektronische) Schaltungsanordnung aufweisen, die mit der jeweiligen Fühlerplatte verbunden ist oder in welche die Fühlerplatte integriert ist und mit der das von der Schmiermittelmenge abhängige Messsignal erzeugbar ist. Bestandteil des Sensors ist folglich nicht nur der Kondensator selbst, sondern eine elektronische Schaltungsanordnung, bei der jedoch auf bekannte Maßnahmen zur Verschaltung von Kondensatoren bzw. Kondensatorplatten im Zuge einer kapazitiven Messung zurückgegriffen werden kann. So kann es sich z. B. bei der Schaltungsanordnung um einen elektronischen Schwingkreis und folglich einen LC-Oszillator handeln, der einerseits von zumindest dem Kondensator (aus Fühlerplatte und Band) und andererseits einer zusätzlichen Spule gebildet wird, wobei die Frequenz des Schwingkreises bzw. des Oszillators als Messsignal von der Kapazität des Kondensators und damit von der innerhalb des Kondensators angeordneten Schmiermittelmenge abhängt. Die durch die elektrische Wirkung des Öls erzeugte Veränderung der Kapazität kann folglich in Form einer Veränderung der Frequenz eines LC-Oszillators erfasst werden, wobei sich die Frequenz des Oszillators verändert, wenn die Ölmenge zwischen Platte und Band kleiner oder größer wird, z. B. kleiner oder größer als die gewünschte Ölmenge wird. Der erfindungsgemäße schmiermittelabhängige bzw. ölabhängige Kondensator wird folglich als frequenzbeeinflussendes Element in einem LC-Oszillator und folglich als frequenzbestimmendes Bauteil eingesetzt. Sofern sich die Ölmenge ändert, ändert sich auch die Frequenz.

Die erste Detektionseinrichtung, die auf kapazitiver Basis arbeitet, kann für die Sensoren mit einer oder mit mehreren (einzelnen) Auswerteeinrichtungen ausgerüstet sein, mit denen durch Auswertung des Messsignals die Schaltungsanordnung ein von dem Messsignal abhängiges und den Schmierzustand repräsentierendes Informationssignal und folglich das Mengensignal der ersten Detektionseinrichtung erzeugbar ist. Dazu kann z. B. in der oder den Auswerteeinrichtungen das Messsignal mit einem oder mit mehreren Vergleichswerten oder mit einem oder mehreren Vergleichsintervallen verglichen werden, wobei durch Vergleich des Messsignals mit der oder den Vergleichswerten bzw. Vergleichsintervallen das Mengensignal erzeugbar ist, z. B. bei Überschreiten oder Unterschreiten eines Vergleichswertes oder eines Vergleichsintervalls. Eine solche Auswerteeinrichtung lässt sich z. B. schaltungstechnisch sehr einfach durch eine grundsätzlich bekannte Filterschaltung realisieren, die bei entsprechender Abweichung ein von der Menge abhängiges Mengensignal erzeugt, das z. B. an eine Meldeeinrichtung weitergeleitet werden kann.

Alternativ zu der beschriebenen Schaltungsanordnung in Form eines LC-Oszillators können auch andere grundsätzlich bekannte Schaltungsanordnungen, z. B. PLL-Schaltungen ("phase locked loop") zum Einsatz kommen.

Insgesamt steht im Vordergrund der beschriebenen kapazitiven, ersten Detektionseinrichtung die Überwachung der Schmiermittelmenge, und zwar bevorzugt breitenselektiv über die Breite des Bandes. Da dieses Messprinzip darauf beruht, dass die Permitivität eines Schmiermittels, z. B. Öls, sehr deutlich von der Permitivität von Luft abweicht, ist zu beobachten, dass sich das Schmiermittel nicht ohne Weiteres von Stoffen mit gleicher oder sehr ähnlicher Permitivität unterscheiden lässt. In einer Presse kommt insbesondere eine Verschmutzung des Schmiermittels mit Holzpartikeln in Betracht und solche Holzpartikel haben eine sehr ähnliche Permitivität wie die üblicherweise eingesetzten flüssigen Schmiermittel, so dass mit einer z. B. kapazitiv arbeitenden ersten Detektionseinrichtung keine Unterscheidung zwischen Schmiermittel und Holz oder auch von mit Holzpartikeln verunreinigten Schmiermitteln erfolgen kann. Aus diesem Grund wird die erste Detektionseinrichtung erfindungsgemäß mit der zweiten Detektionseinrichtung kombiniert.

Die zweite Detektionseinrichtung kann z. B. eine bildgebende Einrichtung aufweisen bzw. als bildgebende Einrichtung ausgebildet sein. Bei einer solchen bildgebenden Einrichtung kann es sich z. B. um eine Kamera handeln, mit der Bilder des Materials auf dem Band aufnehmbar sind. Ferner kann diese zweite Detektionseinrichtung eine Auswerteeinheit, z. B. Rechnereinheit aufweisen, mit der die aufgenommenen Bilder derart auswertbar sind, dass die Art des Materials ermittelbar und gegebenenfalls das Materialsignal erzeugbar ist. Dabei geht die Erfindung von der Erkenntnis aus, dass eine optische Überwachung der Bandoberfläche zwar in der Regel nicht für eine quantitative Mengenauswertung, jedoch gut für eine materialsensitive Auswertung geeignet ist. Denn durch eine geeignete Analyse lässt sich anhand der aufgenommenen Bilder und insbesondere durch Vergleich mit abgespeicherten Bildern, Bilddaten, Mustern oder dergleichen auf Basis geeigneter Algorithmen zwischen einem sauberen Schmiermittel einerseits und einem übermäßig verschmutzten Schmiermittel andererseits oder auch Holzpartikeln, die in einem geschmierten oder auch nicht geschmierten Bereich auf dem Band sein können, unterscheiden. Damit können die im Zusammenhang mit der ersten Detektionseinrichtung gegebenen Nachteile hinsichtlich der fehlenden Materialsensitivität mit der zweiten Detektionseinrichtung kompensiert werden. Die zweite Detektionseinrichtung kann optional eine zusätzliche Lichtquelle aufweisen, mit der die zu analysierende Oberfläche des Bandes gleichmäßig ausgeleuchtet wird. Auf diese Weise können gleichbleibende, standardisierte Lichtverhältnisse für die Aufnahme der Bilder mit der Kamera geschaffen werden. Dieses erhöht die Qualität der Aufnahme und insbesondere die Qualität des Vergleichs mit den hinterlegten Vergleichsdaten bzw. Bildern.

Alternativ können für die erste Detektionseinrichtung und für die zweite Detektionseinrichtung andere Einrichtungen bzw. Analyseverfahren eingesetzt werden.

So kann die erste Detektionseinrichtung, die für die quantitative Analyse bzw. zur Erzeugung eines Mengensignals ausgebildet ist, z. B. als Fluoreszenzmessvorrichtung ausgebildet sein, die zumindest eine elektromagnetische Strahlungsquelle aufweist, mit der elektromagnetische Strahlung eines ersten Wellenlängenbereichs erzeugbar ist und die zumindest einen Sensor aufweist, wobei mit dem Sensor elektromagnetische Strahlung eines zweiten Wellenlängenbereichs, insbesondere Fluoreszenzstrahlung, detektierbar ist, der von dem ersten Wellenlängenbereich abweicht, vorzugsweise oberhalb des ersten Wellenlängenbereichs liegt. Die Erfindung geht bei dieser Art der Detektionseinrichtung von der Erkenntnis aus, dass sich eine quantitative Analyse und folglich eine relative Mengenbestimmung des Schmiermittels auch durch die Messung von Fluoreszenzstrahlung realisieren lässt, die von dem Schmiermittel, das mit elektromagnetischer Strahlung bestrahlt wird, emittiert wird. So lässt sich z. B. die zu analysierende Oberfläche mit Wellenlänge in einem Wellenlängenbereich von 190 bis 420 nm, insbesondere im Bereich von 250 nm bis 420 nm und besonders bevorzugt von 300 nm bis 365 nm bestrahlen. Diese Bestrahlung führt aufgrund der Schmiermittelbestandteile bzw. spezieller Schmiermitteladditive zur Anregung der atomaren bzw. molekularen Zustände, gefolgt von spontaner Emission von elektromagnetischer Strahlung, die energieärmer ist und folglich größere Wellenlängen als die zuvor absorbierte Strahlung aufweist. Insofern wird der Sensor dieser Fluoreszenzmessvorrichtung bevorzugt so eingerichtet, dass die Strahlung in dem gewünschten Wellenlängenbereich zuverlässig und effektiv detektiert wird, z. B. in einem Wellenlängenbereich von 420 nm bis 575 nm, insbesondere 420 nm bis 520 nm, besonders bevorzugt 440 nm bis 495 nm. Insgesamt lässt sich auch durch eine solche Floreszenzanalyse der Schmiermittelfilm quantitativ analysieren und folglich ein Mengensignal erzeugen, das die Schmiermittelmenge repräsentiert bzw. das eine Überschreitung oder Unterschreitung eines zulässigen Schmiermittelintervalls repräsentiert.

Sofern eine solche Fluoreszenzmessvorrichtung als erste Detektionseinrichtung eingesetzt wird, wird wiederum eine zweite Detektionseinrichtung eines anderen Typs eingesetzt, z. B. die beschriebene kamerabasierte bildgebende Einrichtung, mit der wiederum Rückschlüsse auf die Art des Materials gezogen werden können.

Es liegt jedoch optional auch im Rahmen der Erfindung, eine Fluoreszenzmessvorrichtung des beschriebenen Typs als zweite Detektionseinrichtung einzusetzen, die für eine Unterscheidung der auf dem Band befindlichen Materialien eingesetzt wird. Denn durch Analyse der emittierten Fluoreszenzstrahlung können bei geeigneter Ausgestaltung der Sensoren Rückschlüsse auf die Art des Materials erfolgen, und zwar insbesondere dann, wenn dem Schmiermittel spezielle Additive beigegeben sind oder beigegeben werden, die in der emittierten Fluoreszenzstrahlung eine Unterscheidung des Öls von z. B. Holzpartikeln oder anderen Schmutzpartikeln ermöglichen. In diesem Zusammenhang besteht z. B. die Möglichkeit, mit mehreren Sensoren die emittierte Fluoreszenzstrahlung in unterschiedlichen Frequenzbereichen bzw. Wellenlängenbereichen aufzunehmen, um Unterschiede zwischen verschiedenen Materialien zu ermitteln.

Die erste Detektionseinrichtung kann in einer weiteren Ausführungsform auch als Interferenzmessvorrichtung ausgebildet sein, die zumindest eine elektromagnetische Strahlungsquelle, z. B. eine Röntgenquelle aufweist und die zumindest einen Sensor aufweist, z. B. eine CCD-Kamera. Mit diesem Sensor lässt sich z. B. ein Interferenzmuster aufnehmen, das aufgrund der am Band reflektierten Strahlung und der durch das auf dem Band befindliche Material gebeugten Strahlung entsteht. Es kommt zu charakteristischen Interferenzen, aus denen durch geeignete Auswertung die Schmiermittelmenge ermittelt bzw. auf die Schmiermittelmenge geschlossen werden kann. Insofern eignet sich eine solche Messmethode für den Einsatz in der ersten Detektionseinrichtung. Kombiniert werden kann eine solche Detektionseinrichtung wiederum mit den bereits erwähnten Maßnahmen für die zweite, materialsensitive Detektionseinrichtung.

Optional liegt es jedoch auch im Rahmen der Erfindung, eine Interferenzmessvorrichtung als zweite Detektionseinrichtung für eine materialsensitive Auswertung einzusetzen.

Die Erfindung betrifft im Übrigen nicht nur eine Vorrichtung zur Überwachung des Schmierzustandes, sondern auch ein entsprechendes Verfahren zur Überwachung eines mit einem Schmiermittel beaufschlagten, umlaufenden Bandes für den Transport von Pressgut, insbesondere in einer kontinuierlich arbeitenden Presse, wobei mit zumindest einer im Bereich des Bandes angeordneten ersten Detektionseinrichtung eines ersten Typs und mit zumindest einer im Bereich des Bandes angeordneten zweiten Detektionseinrichtung eines zweiten Typs zumindest ein Signal erzeugt wird. Dieses Verfahren ist dadurch gekennzeichnet, dass mit der ersten Detektionseinrichtung ein von der Menge des auf dem Band befindlichen Materials abhängiges Mengensignal erzeugt wird und dass mit der zweiten Detektionseinrichtung ein von der Art des auf dem Band befindlichen Materials abhängiges Materialsignal erzeugt wird und dass aus dem Materialsignal oder durch Auswertung einer Kombination aus Materialsignal und Mengensignal zwischen einem auf dem Band befindlichen sauberen Schmiermittel einerseits und auf dem Band befindlichen Schmutzpartikeln oder einem mit Schmutzpartikeln verunreinigten Schmiermittel andererseits unterschieden wird.

Ein solches Verfahren kann in vorteilhafter Weise unter Verwendung einer erfindungsgemäßen Vorrichtung durchgeführt werden.

Gegenstand der Erfindung ist außerdem eine kontinuierlich arbeitende Presse, die zumindest ein oberes endlos umlaufendes Band (Pressband) und ein unteres endlos umlaufendes Band (Pressband) aufweist, wobei das Pressgut durch den Pressspalt zwischen dem oberen und dem unteren Band hindurchgeführt wird. Bevorzugt handelt es sich um eine Pressvorrichtung, bei der das Pressgut unter Anwendung von Druck und/oder Wärme zu einer Platte bzw. einem endlosen Plattenstrang verpresst wird. Alternativ kann es sich bei einer solchen Presse auch um eine Vorwärmeinrichtung handeln, die z. B. in einem kontinuierlichen Pressprozess der Vorwärmung des Pressgutes dient, bevor dieses in eine kontinuierlich arbeitende Presse zur Herstellung der eigentlichen Platte einläuft. Bevorzugt werden solche kontinuierlich arbeitenden Pressen nach Art einer Doppelbandpresse erfasst, bei denen die Pressbänder unter Zwischenschaltung von Wälzkörpern, z. B. von Rollstangen oder Rollstäben, an der jeweiligen Pressenplatte abgestützt sind. Die Erfindung betrifft jedoch alternativ auch Pressen mit umlaufenden Pressbändern anderer Bauart, z. B. Kalanderpressen. Stets ist eine solche Presse mit einer Vorrichtung zur Überwachung des Schmierzustandes der beschriebenen Art ausgerüstet, d.h., die Erläuterungen zur Schmierung und Überwachung des Bandes betreffen bei einer Presse bevorzugt das jeweilige obere Pressband und/oder das jeweilige untere Pressband. Gegenstand einer solchen Presse ist in der Regel auch eine Auftragsvorrichtung, mit der ein Schmiermittel auf das obere und/oder das untere Band (unmittelbar oder mittelbar) aufbringbar ist. Die erfindungsgemäße Presse ist folglich dadurch gekennzeichnet, dass die Presse mit einer Vorrichtung zur Überwachung des Schmierzustandes der beschriebenen Art ausgerüstet ist.

In entsprechender Weise betrifft die Erfindung schließlich auch ein Verfahren zum Betrieb einer derartigen Presse, das dadurch gekennzeichnet ist, dass der Schmierzustand der Presse mit einem erfindungsgemäß ausgestalteten Verfahren zur Überwachung eines mit einem Schmiermittel beaufschlagten, umlaufenden Bandes für den Transport von Pressgut überwacht wird.

Im Folgenden wird die Erfindung anhand einer lediglich ein Ausführungsbeispiel darstellenden Zeichnung näher erläutert. Es zeigen
- Fig. 1: schematisch vereinfacht eine kontinuierlich arbeitende Presse mit einer Vorrichtung zur Überwachung des Schmierzustandes der Pressbänder,
- Fig. 2: schematisch vereinfacht eine Vorrichtung zur Überwachung des Schmierzustandes innerhalb einer Presse nach Fig. 1 in einer ersten Ausführungsform,
- Fig. 3: eine Schaltungsanordnung der ersten Detektionseinrichtung einer Vorrichtung nach Fig. 2,
- Fig. 4: schematisch vereinfacht eine Vorrichtung nach Fig. 2 in einer abgewandelten, zweiten Ausführungsform, und
- Fig. 5: eine dritte Ausführungsform der Erfindung.

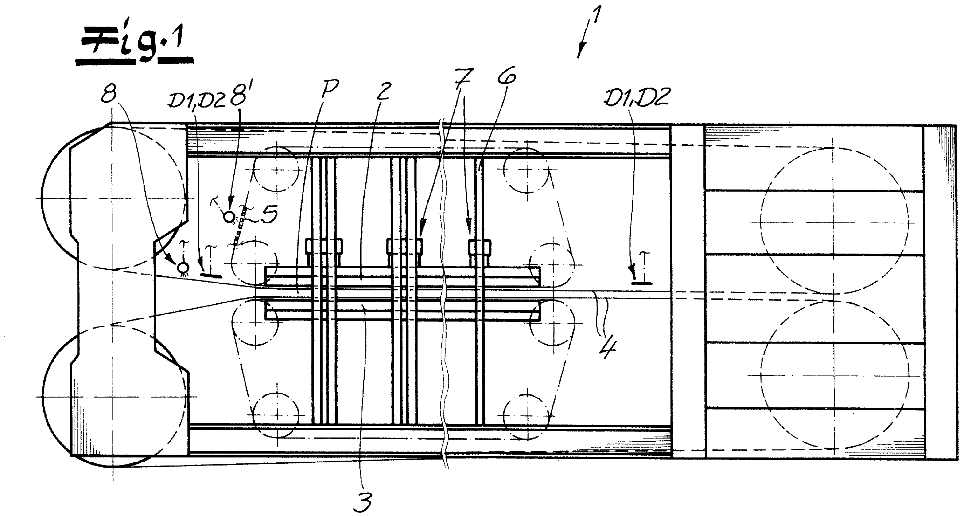

In Fig. 1 ist eine kontinuierlich arbeitende Presse 1 dargestellt, die z. B. zum Verpressen von Pressgutmatten zu Pressgutplatten im Zuge der Herstellung von Spanplatten, Faserplatten oder anderen Holzwerkstoffplatten geeignet und bestimmt ist. Die kontinuierliche Presse 1 weist in ihrem grundsätzlichen Aufbau ein Pressenoberteil mit einer oberen beheizbaren Pressenplatte 2 und ein Pressenunterteil mit einer unteren beheizbaren Pressenplatte 3 auf. Die beheizbaren Pressenplatten 2, 3 werden auch als Heizplatten bezeichnet. Im Pressenoberteil sowie im Pressenunterteil sind endlos umlaufende Bänder 4 geführt, die als Pressbänder 4 bezeichnet werden und z. B. aus Stahl gefertigt sind. Zwischen den Pressbändern 4 wird ein Pressspalt P gebildet, durch den das Pressgut hindurchgeführt wird. Die Pressbänder 4 sind unter Zwischenschaltung von Rollstäben bzw. Rollstangen 5 gegen die Pressenplatten 2, 3 abgestützt. Die Rollstäbe 5 sind dabei z. B. an Rollstabketten geführt, die nicht im Detail dargestellt sind. Das Pressgut wird innerhalb der Presse unter Anwendung von Druck und Wärme zu einem aus der Presse auslaufenden Plattenstrang verpresst. Die dazu erforderlichen Presskräfte werden mit Kraftmitteln, insbesondere mit hydraulischen Presszylindern 7 aufgebracht, mit denen z. B. die obere Pressenplatte 2 oder die untere Pressenplatte 3 beaufschlagt wird. Diese Presszylinder 7 sind am Pressengestell, z. B. an dessen Pressenrahmen 6 abgestützt. Die Fig. 1 zeigt dabei lediglich beispielhaft eine Oberkolbenpresse, bei der die obere Pressenplatte 2 mit den Presszylindern 7 beaufschlagt wird.

Die Pressbänder werden mit einem Schmiermittel beaufschlagt, insbesondere mit einem flüssigen Schmiermittel, z. B. mit Öl. Dabei ist in Fig. 1 die Möglichkeit angedeutet, das jeweilige Pressband unmittelbar mit dem Schmiermittel zu beaufschlagen, z. B. durch eine geeignete Auftragsvorrichtung 8, die z. B. als sich quer zur Bandlaufrichtung über die Bandbreite erstreckende Bedüsungsvorrichtung ausgebildet sein kann. Alternativ oder ergänzend kann jedoch eine in Fig. 1 ebenfalls angedeutete Auftragsvorrichtung 8' vorgesehen sein, die die umlaufenden Rollstangen 5 beaufschlagt, so dass über die Rollstangen eine mittelbare Beaufschlagung des Pressbandes mit dem Schmiermittel erfolgt. Dabei zeigt Fig. 1 lediglich die Schmierung des oberen Bandes. Selbstverständlich sind entsprechende Maßnahmen auch im Bereich des unteren Bandes vorgesehen. Stets erfolgt die Beaufschlagung der dem Pressgut abgewandten "inneren" Oberfläche des jeweiligen Pressbandes 4.

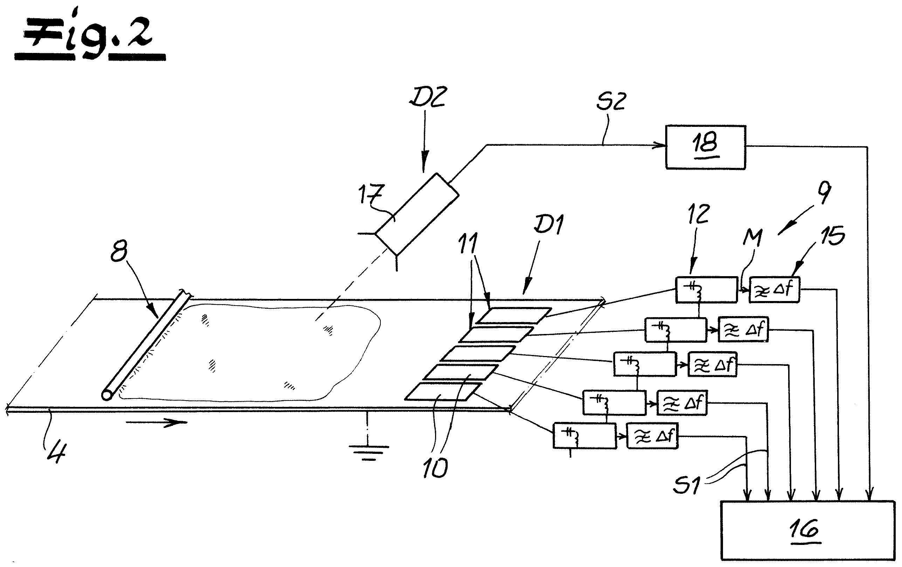

Die Presse 1 ist mit einer Vorrichtung zur Überwachung des Schmierzustandes der mit dem Schmiermittel beaufschlagten, umlaufenden Bänder ausgerüstet. Diese Vorrichtung ist beispielhaft in Fig. 2 dargestellt. Sie weist einerseits eine erste Detektionseinrichtung D1 und andererseits eine zweite Detektionseinrichtung D2 auf. Die erste Detektionseinrichtung D1 ist dazu eingerichtet, ein von der Menge des auf dem Band 4 befindlichen Materials abhängiges Mengensignal S1 zu erzeugen. Zur Unterscheidung eines auf dem Band 4 befindlichen Schmiermittels von auf dem Band 4 befindlichen Schmutzpartikeln oder von einem mit Schmutzpartikeln verunreinigten Schmiermittel ist die zweite Detektionseinrichtung D2 dazu eingerichtet, ein von der Art des auf dem Band 4 befindlichen Materials abhängiges Materialsignal S2 zu erzeugen. Durch Kombination zweier unterschiedlicher Messeinrichtungen bzw. zweier unterschiedlicher Messverfahren lässt sich folglich nicht nur zuverlässig die Schmiermittelmenge auf dem Band überwachen, sondern es kann auch eine Unterscheidung zwischen unterschiedlichen Materialien erfolgen. Damit kann z. B. zuverlässig vermieden werden, dass bei einer quantitativen Überwachung des Schmiermittels bezogen auf die Menge des Schmiermittels fehlerhafte Rückschlüsse gezogen werden, wenn sich die gemessene Menge nicht auf ein Schmiermittel, sondern auf Verschmutzungen, z. B. Holzpartikel oder dergleichen bezieht. Insofern kann die erste Detektionseinrichtung D1 gezielt auf eine mengenmäßige Erfassung ausgelegt sein, ohne dass eine Unterscheidung zwischen z. B. einem Schmiermittel und Holzpartikeln erfolgen muss. In dem Ausführungsbeispiel nach Fig. 2 ist die erste Detektionseinrichtung D1 als kapazitiv arbeitende Vorrichtung ausgebildet. Sie weist mehrere im Bereich des Bandes angeordnete und über die Bandbreite verteilte Sensoren 9 auf. Diese Sensoren 9 sind als kapazitiv arbeitende Sensoren ausgebildet, die jeweils zumindest eine in einem Abstand d zum Band 4 angeordnete Fühlerplatte 10 aufweisen, die mit dem Band 4 jeweils einen Kondensator 11 bilden, dessen Kapazität von der auf dem Band angeordneten Schmiermittelmenge abhängt. Dabei ist in Fig. 2 erkennbar, dass über die Breite des Bandes 4 nebeneinander mehrere Fühlerplatten 10 angeordnet sind, die mit dem Band gemeinsam mehrere nebeneinander angeordnete Kondensatoren 11 bilden, die unterschiedlichen Breitenpositionen des Bandes zugeordnet sind. Im Ausführungsbeispiel sind fünf nebeneinander angeordnete Fühlerplatten 10 und folglich fünf nebeneinander angeordnete Sensoren dargestellt, so dass eine Überwachung des Schmierzustandes in fünf einzelnen Spuren auf dem Band möglich ist.

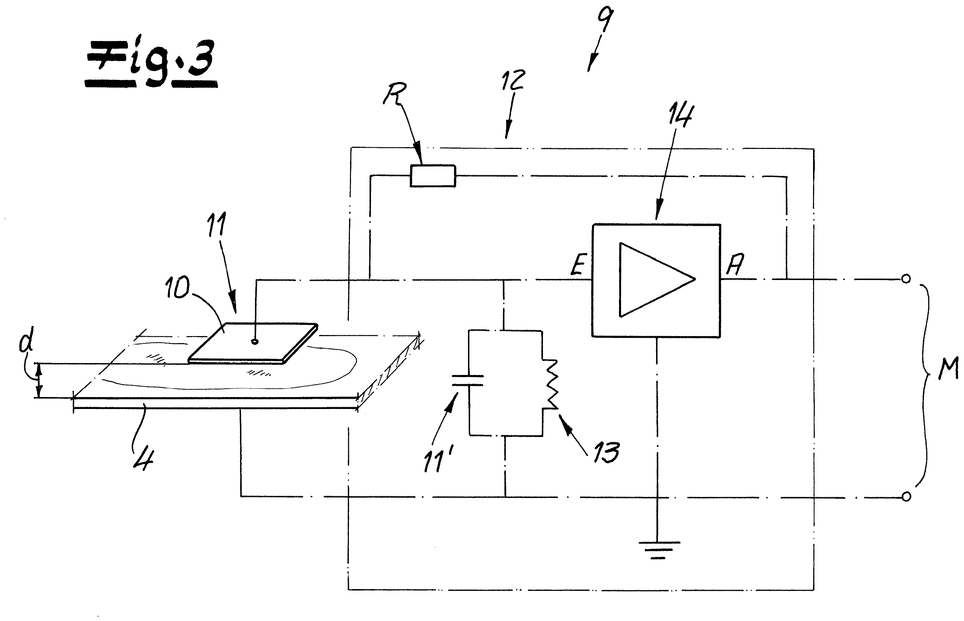

Die Sensoren 9 weisen jeweils eine Schaltungsanordnung 12 auf, die mit der jeweiligen Fühlerplatte 10 verbunden ist bzw. in welche die jeweilige Fühlerplatte 10 integriert ist (vgl. z. B. Fig. 3). Mit dieser Schaltungsanordnung 12 kann (für die jeweilige Breitenposition) ein von der Schmiermittelmenge abhängiges Messsignal M erzeugt werden. In dem dargestellten Ausführungsbeispiel ist diese Schaltungsanordnung 12 als Schwingkreis und folglich als LC-Oszillator ausgebildet, welche einerseits den Kondensator 11 und andererseits eine Spule 13 aufweisen. In Fig. 3 ist dabei eine Variante angedeutet, bei der zunächst ein Basisschwingkreis mit der Spule 13 und einem unmittelbar zugeordneten Kondensator 11' vorgesehen ist, dem der Kondensator 11 aus Fühlerplatte 10 und Band 4 parallel geschaltet ist, so dass sich die Kapazitäten aus 11 und 11' addieren. Ferner sind ein Verstärker 14 mit Eingang E und Ausgang A sowie ein Widerstand R dargestellt. Der Ausgang dieser Schaltungsanordnung 12 gemäß Fig. 3 liefert ein Frequenzsignal als Messsignal, d.h., die Frequenz des Schwingkreises hängt von der Kapazität des Kondensators 11 und damit von der innerhalb des Kondensators 11 auf dem Band 4 angeordneten Schmiermittelmenge ab. Die Frequenz des Oszillators verändert sich folglich, wenn die Ölmenge zwischen Platte und Band kleiner oder größer wird. Der schmiermittelabhängige Kondensator 11 wird folglich in einem LC-Oszillator als frequenzbestimmendes Bauteil eingesetzt, so dass die Frequenz als Messsignal M zur Verfügung steht.

Die Sensoren 9 (bzw. deren Schaltungen 12) sind mit einer oder mit mehreren Auswerteeinrichtungen 15 verbunden, die im Ausführungsbeispiel als Filterschaltungen 15 ausgebildet sind. Sie sind in den Figuren schematisch vereinfacht als getrennte Komponenten dargestellt, können jedoch selbstverständlich auch schaltungstechnisch untereinander und/oder mit den Schaltungen 12 kombiniert sein. Mit den Auswerteeinrichtungen 15 werden durch Auswertung des Messsignals jeweils die von dem Messsignal M abhängigen und den Schmierzustand repräsentierenden Mengensignal S1 erzeugt. Dazu können die Messsignale M in den Auswerteeinrichtungen 15 jeweils mit einem oder mit mehreren Vergleichswerten oder mit einem Vergleichsintervall verglichen werden und in Abhängigkeit von dem Ergebnis des Vergleichs kann das Informationssignal S1 erzeugt werden, z. B. bei Überschreiten oder bei Unterschreiten eines Vergleichswertes oder eines Vergleichsintervalls. So besteht z. B. die Möglichkeit, dass ein Informationssignal S1 erzeugt und an eine Meldeeinrichtung 16 übermittelt wird, wenn für die jeweilige Breitenposition eine Unterschmierung oder eine Überschmierung festgestellt wird. Schaltungstechnisch lässt sich dieses z. B. durch eine Filterschaltung realisieren, d.h., das Informationssignal wird erzeugt, wenn die gemessene Frequenz von einer vorgegebenen Frequenz abweicht oder aus einem vorgegebenen Frequenzbereich fällt.

Insgesamt ist die erste Detektionseinrichtung gemäß Fig. 2 auf die mengenmäßige Überwachung des Schmiermittels ausgelegt. Beispielsweise mit dem beschriebenen kapazitiven Prinzip lässt sich jedoch gegebenenfalls nicht erkennen, ob sich die innerhalb des Kondensators angeordnete Materialmenge tatsächlich auf ein (sauberes) Schmiermittel bezieht oder auf ein mit Holzpartikeln verschmutztes Schmiermittel oder gar auf eine Schicht aus Holzpartikeln. Denn Holzpartikel einerseits und das Schmiermittel andererseits beeinflussen die Permitivität des Kondensators in etwa in gleicher Weise.

Aus diesem Grund ist erfindungsgemäß die in Fig. 2 ebenfalls dargestellte zweite Detektionseinrichtung D2 vorgesehen, die hier eine bildgebende Einrichtung, nämlich eine Kamera 17 sowie eine Rechnereinheit 18 aufweist. Mit der Kamera können ein oder mehrere Bilder des Materials auf dem Band 4 aufgenommen werden, die anschließend mit der Rechnereinheit 18 derart ausgewertet werden, dass die Art des Materials ermittelt wird bzw. dass ein Materialsignal S2 erzeugt wird, das eine Information darüber enthält, ob es sich bei dem Material um ein Schmiermittel in der gewünschten Zusammensetzung handelt oder um ein übermäßig mit Verschmutzungen verschmutztes Schmiermittel oder gar um Holzpartikel. Dabei kann die Anzahl der Bilder pro Zeiteinheit der Geschwindigkeit des Pressbandes im Betrieb angepasst sein. Das erzeugte Materialsignal S2 wird ebenfalls der Melde- und Rechnereinheit 16 zugeführt, die auch die Mengensignale S1 erhält (vgl. Fig. 2). Optional kann die materialsensitive Analyse mit der zweiten Detektionseinrichtung D2 und für verschiedene Breitenbereiche bzw. Spuren erfolgen, die den von der ersten Detektionseinrichtung D1 erzeugten Spuren entsprechen. Diese Option ist nicht dargestellt.

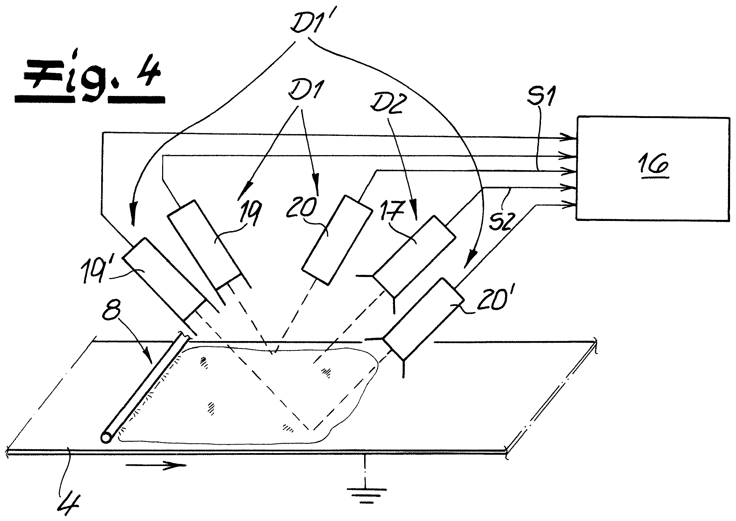

Fig. 4 zeigt eine zweite Ausführungsform der Erfindung, bei der die erste Detektionseinrichtung D1 für die Erzeugung des Mengensignals S1 als Fluoreszenzmessvorrichtung ausgebildet ist. Diese weist eine elektromagnetische Strahlungsquelle 19, z. B. einen Laser, auf, der elektromagnetische Strahlung in einem ersten Wellenlängenbereich erzeugt und damit die Oberfläche des Bandes bestrahlt. Ferner ist ein Sensor 20 vorgesehen, der Fluoreszenzstrahlung ermittelt, die aus der auf dem Band befindlichen Schmiermittelschicht emittiert wird und die eine größere Wellenlänge und folglich geringere Frequenz aufweist als die eingestrahlte Laserstrahlung. Mit Hilfe einer solchen Fluoreszenzmessung lässt sich durch geeignete Auswertung mit dem Rechnersystem 16 ebenfalls auf die Schmiermittelmenge schließen. Diese erste Detektionseinrichtung D1 wird wiederum mit der Detektionseinrichtung D2 kombiniert, die bereits im Zusammenhang mit Fig. 2 erläutert wurde.

Optional (oder ergänzend) zu der ersten Detektionseinrichtung D1 als Fluoreszenzmessvorrichtung kann die erste Detektionseinrichtung D1 auch als Interferenzmessvorrichtung ausgebildet sein. Auch eine solche ist in Fig. 4 dargestellt. Es sind einerseits eine Röntgenquelle 19' und andererseits ein Sensor 20' erkennbar, die wiederum mit dem Rechnersystem verbunden sind. Mit dem Sensor 20', der z. B. als CCD-Kamera ausgebildet sein kann, kann ein Interferenzmuster aufgenommen werden, das ebenfalls Informationen über die Schmiermittelmenge enthält und insofern mit der ersten Detektionseinrichtung D1 kombinierbar ist.

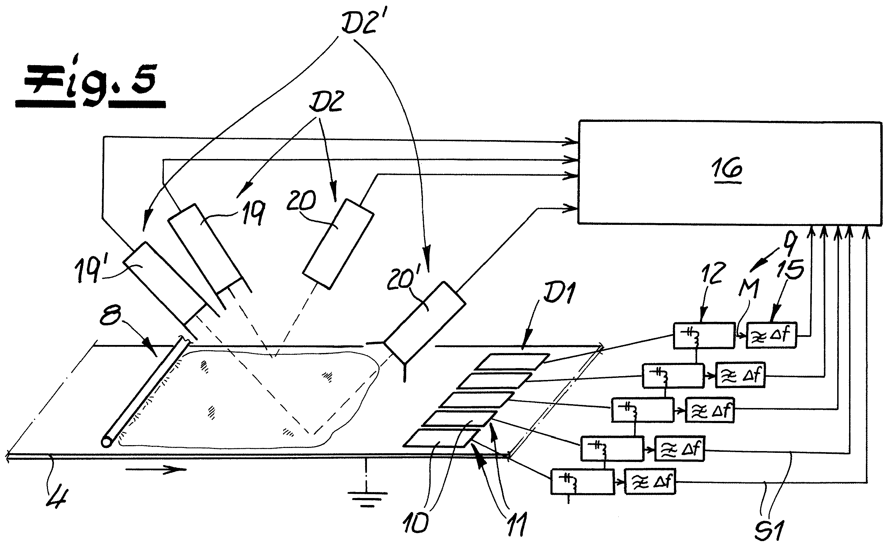

In Fig. 5 ist eine dritte Ausführungsform der Erfindung dargestellt, bei der die erste Detektionseinrichtung D1 wiederum als kapazitive Einrichtung ausgebildet ist, die bereits im Zusammenhang mit Fig. 2 erläutert wurde. In diesem Fall kann die zweite Detektionseinrichtung, die für die Materialanalyse verantwortlich ist, entweder als Fluoreszenzmessvorrichtung D2 oder als Interferenzmessvorrichtung D2` ausgebildet sein.

## Patentansprüche

1. Kontinuierlich arbeitende Presse (1), mit einem oberen endlos umlaufenden Band (4) und einem unteren endlos umlaufenden Band (4),
wobei Pressgut durch den Pressspalt zwischen dem oberen und dem unteren Band (4) hindurchgeführt und unter Anwendung von Druck und/oder Wärme zu einer Platte oder einem endlosen Plattenstrang verpresst wird,
und mit zumindest einer Auftragsvorrichtung (8, 8'), mit der ein Schmiermittel auf das obere Band (4) und/oder auf das untere Band (4) unmittelbar oder mittelbar aufbringbar ist,
wobei die Presse (1) mit einer Vorrichtung zur Überwachung des Schmierzustandes eines mit dem Schmiermittel beaufschlagten, umlaufenden Bandes (4) ausgerüstet ist,
wobei die Vorrichtung zumindest eine im Bereich des Bandes (4) angeordnete erste Detektionseinrichtung (D1) eines ersten Typs und
zumindest eine im Bereich des Bandes (4) angeordnete zweite Detektionseinrichtung (D2) eines zweiten Typs aufweist,
**dadurchgekennzeichnet,**
dass die erste Detektionseinrichtung (D1) dazu eingerichtet ist, ein von der Menge des auf dem Band (4) befindlichen Materials abhängiges Mengensignal zu erzeugen und
dass zur Unterscheidung eines auf dem Band (4) befindlichen Schmiermittels von auf dem Band (4) befindlichen Schmutzpartikeln oder von einem mit Schmutzpartikeln verunreinigten Schmiermittel die zweite Detektionseinrichtung (D2) dazu eingerichtet ist, ein von der Art des auf dem Band (4) befindlichen Materials abhängiges Materialsignal (S2) zu erzeugen.

2. Presse nach Anspruch 1, **gekennzeichnet durch** ein Melde- und/oder Rechnersystem (16), mit dem das Mengensignal (S1) und das Materialsignal (S2) anzeigbar sind und/oder mit dem das Mengensignal (S1) und das Materialsignal (S2) derart auswertbar sind, dass aus dem Materialsignal (S2) oder aus einer Kombination aus Mengensignal (S1) und Materialsignal (S2) die Art des auf dem Band befindlichen Materials ermittelbar ist.

3. Pressenach Anspruch 1 oder 2, **dadurch gekennzeichnet, dass** die erste Detektionseinrichtung (D1) einen oder mehrere kapazitiv arbeitende Sensoren (9) aufweist, die jeweils zumindest eine in einem Abstand zum Band (4) angeordnete Fühlerplatte (10) aufweisen, die mit dem Band jeweils einen Kondensator (11) bilden, dessen Kapazität von der auf dem Band (4) angeordneten Menge des Materials, insbesondere Schmiermittels, abhängt.

4. Presse nach einem der Ansprüche 1 bis 3, **dadurch gekennzeichnet, dass** die zweite Detektionseinrichtung (D2) eine bildgebende Einrichtung, z. B. eine Kamera (17), und eine Rechnereinheit (18) aufweist, wobei mit der Kamera ein oder mehrere Bilder des Materials auf dem Band aufnehmbar sind, welche mit der Rechnereinheit (18) derart auswertbar sind, dass die Art des Materials ermittelbar und gegebenenfalls das Materialsignal (S2) erzeugbar ist.

5. Presse nach Anspruch 4, **dadurch gekennzeichnet, dass** in der Rechnereinheit (18) der zweiten Detektionseinrichtung (D2) Informationen, z. B. Bilddaten, Muster oder dergleichen verschiedener Materialien, insbesondere des Schmiermittels einerseits und von Schmutzpartikeln und/oder eines mit Schmutzpartikeln verunreinigten Schmiermittels andererseits gespeichert sind und dass die mit der Kamera (17) aufgenommenen Bilder durch Vergleich mit den gespeicherten Informationen auswertbar sind.

6. Presse nach Anspruch 4 oder 5, **dadurch gekennzeichnet, dass** die zweite Detektionseinrichtung (D2) eine zusätzliche Lichtquelle aufweist, mit der die zu analysierende Oberfläche des Bandes (4) gleichmäßig ausleuchtbar ist, um gleichbleibende, standardisierende Lichtverhältnisse für die Aufnahme der Bilder mit der Kamera (17) zu schaffen.

7. Presse nach einem der Ansprüche 1 bis 6, **dadurch gekennzeichnet, dass** die erste Detektionseinrichtung (D1) oder die zweite Detektionseinrichtung (D2) als Fluoreszenzmessvorrichtung ausgebildet ist, die zumindest eine elektromagnetische Strahlungsquelle (19) aufweist, mit der elektromagnetische Strahlung eines ersten Wellenlängenbereichs erzeugbar ist und die zumindest einen Sensor (20) aufweist, wobei mit dem Sensor elektromagnetische Strahlung eines zweiten Wellenlängenbereichs, insbesondere Fluoreszenzstrahlung, detektierbar ist, der von dem ersten Wellenlängenbereich abweicht, vorzugsweise oberhalb des ersten Wellenlängenbereichs liegt.

8. Presse nach einem der Ansprüche 1 bis 7, **dadurch gekennzeichnet, dass** die erste Detektionseinrichtung (D1) oder die zweite Detektionseinrichtung (D2) als Interferenzmessvorrichtung ausgebildet ist, die zumindest eine elektromagnetische Strahlungsquelle (19), z. B. eine Röntgenquelle, aufweist und die zumindest einen Sensor (20`), z. B. eine CCD-Kamera, aufweist, wobei mit dem Sensor ein Interferenzmuster des an dem Band (4) und dem auf dem Band (4) befindlichen Material reflektierten und gebeugten Strahlung detektierbar ist.

9. Verfahren zum Betrieb einer Presse nach einem der Ansprüche 1 bis 8, wobei der Schmierzustand eines mit einem Schmiermittel beaufschlagten umlaufenden Bandes der Presse mit einem Verfahren überwacht wird, bei welchem mit zumindest einer im Bereich des Bandes (4) angeordneten ersten Detektionseinrichtung (D1) eines ersten Typs und mit zumindest einer im Bereich des Bandes (4) angeordneten zweiten Detektionsvorrichtung (D2) eines zweiten Typs zumindest ein Signal erzeugt wird,
**dadurch gekennzeichnet, dass** mit der ersten Detektionseinrichtung (1) ein von der Menge des auf dem Band befindlichen Materials abhängiges Mengensignal (S1) erzeugt wird und
dass mit der zweiten Detektionseinrichtung (D2) ein von der Art des auf dem Band befindlichen Materials abhängiges Materialsignal (S2) erzeugt wird und
dass aus dem Materialsignal (S2) oder durch Auswertung einer Kombination aus Materialsignal (S2) und Mengensignal (S1) zwischen einem auf dem Band (4) befindlichen sauberen Schmiermittel einerseits und auf dem Band (4) befindlichen Schmutzpartikeln oder einem mit Schmutzpartikeln verunreinigten Schmiermittel andererseits unterschieden wird.

10. Verfahren nach Anspruch 9, dass das Materialsignal (S2) mit einer bildgebenden Einrichtung, z. B. einer Kamera (17), und einer Rechnereinheit (18) erzeugt wird, vorzugsweise durch Vergleich von mit der Kamera (17) aufgenommenen Bildern mit in der Rechnereinheit (18) gespeicherten Vergleichsinformationen, z. B. Vergleichsbildern oder Vergleichsmustern.
